(19) Europäisches Patentamt
European Patent Office
Office européen des brevets

(11) **EP 3 131 026 A1**

(12) **EUROPEAN PATENT APPLICATION**

(43) Date of publication:
**15.02.2017 Bulletin 2017/07**

(51) Int Cl.:
***G06F 19/00*** *(2011.01)*

(21) Application number: **15180838.3**

(22) Date of filing: **12.08.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Institute of Mathematics and Computer Science,**
**University of Latvia**
**1459 Riga (LV)**

(72) Inventors:
• **BARZDINS, Janis Visvaldis**
**1459 Riga (LV)**

• **CERANS, Karlis**
**1459 Riga (LV)**
• **GRASMANIS, Mikus**
**1459 Riga (LV)**
• **RENCIS, Edgars**
**1459 Riga (LV)**
• **PODNIEKS, Karlis**
**1459 Riga (LV)**
• **SOSTAKS, Agris**
**1459 Riga (LV)**

(74) Representative: **Kuzjukevica, Lucija**
**Petersona Patents, SIA**
**P.O. Box 61**
**1010 Riga (LV)**

(54) **A SYSTEM AND METHOD FOR INSTANTANEOUS AD HOC QUERYING OF HOSPITAL INFORMATION**

(57) The present invention relates to a system and a method for instantaneous *ad hoc* querying of hospital information to support organizational decision-making activities in hospitals. The system is able to respond to a query in less than one second having a database of millions to tens of millions of records.

A system for *ad hoc* querying comprises a database configured to store a data into granules, an input device and an output device for user interface, and a processor configured to process queries.

A method comprising the steps of: collecting and storing hospital information; re-designing the database to obtain a granular ontology, granulating data according to a granular ontology; generating a library of API functions; developing an adjusted query language; translating query language into sequences of applications of the API; and - processing of queries in order to give a queried answer.

Fig. 1

**Description**

Technical Field

The present invention relates to a system and a method for instantaneous *ad hoc* querying of hospital information to support organizational decision-making activities of managers in hospitals, especially in large hospitals.

Background Art

If we look at traditional hospital information systems that use relational databases primarily, routine tasks (such as entering information, obtaining various reports in predefined formats etc.) are solved quite well usually. However, if a hospital manager wants to perform a deeper search and obtain an answer to some *ad hoc* query, then, typically, he must ask a programmer. Moreover, managers firstly have to engage in an interaction with the programmer in order to understand, what information can be found in the hospital databases. He usually cannot obtain this information from the entity relationship (ER) model, since it is technical and designed only for the use of information technology specialists. Three problems therefore arise in this context:

1) how to depict the data ontology to be easily understandable by hospital managers as domain experts;
2) how to develop a query language based on this representation of the data ontology, such that a manager, also known as domain expert, could formulate queries themselves and understand its answers;
3) how to implement the query language efficiently enough in order to get the answer to a sufficiently wide class of queries in a reasonable time - of around one second. The volume of data can be millions to tens of millions of records.

These three problems are, of course, mutually correlated. For example, we can provide a very rich query language that solves the second problem, but it will not be efficiently implementable. Or we can offer a data ontology that is easily understandable for domain experts, but it will not cover a sufficiently wide part of the domain to be able to formulate interesting questions about it. Therefore, a more general problem arises: how to find a *balanced* solution for the three above-mentioned particular problems so that the potential end-users are satisfied.

One of the very traditional trends towards the "3How" problem is Microsoft Excel, i.e. domain experts can order a programmer to prepare the database data in the form of MS Excel spreadsheet, which is often familiar to them. However, there are some restrictions in MS Excel, when the volume of data grows. The response time of the system can become quite large in case of big data or data of a more complicated structure. Besides, if the query itself becomes more complicated, for a non-programmer, it may be difficult to formulate it at all.

There are a lot of works related to the "3How" problem (sometimes named differently) in the medical area. Good survey of this kind of work can be found in publication of Ping, X.O. et al (Ping, X.O., Chung, Y., et al: A Web-Based Data-Querying Tool Based on Ontology-Driven Methodology and Flowchart-Based Model, JMIR Medical Informatics, Vol. 1, No. 1, 2013 [doi: 10.2196/medinform.2519]).

All these works are based on the thesis that a direct usage of low-level query language such as SQL is not suitable for experts of medical domain. These works therefore propose domain-specific query building methods and tools to assist users in building and executing database queries. Roughly speaking two approaches for building such tools are studied. The first approach proposes building data querying models based on workflows which is described in publication of Huser, V. et al (Huser, V., Narus, S.P., Rocha, R.A.: Evaluation of a flowchart-based EHR query system: a case study of RetroGuide. J Biomed Inform 2010 Feb; 43(1), pp. 41-50 [doi: 10.1016/j.jbi.2009.06.001]). The other direction offers finding query patterns for clinical research described in papers of Austin, T. et al (Austin, T., Kalra, D., Tapuria, A., Lea, N., Ingram, D.: Implementation of a query interface for a generic record server. Int J Med Inform 2008 Nov; 77(11), pp. 754-764. [doi: 10.1016/j.ijmedinf.2008.05.003]) and Mabotuwana, T. et al (Mabotuwana, T., Warren, J.: An ontology-based approach to enhance querying capabilities of general practice medicine for better management of hypertension. Artif Intell Med 2009 Oct; 47(2), pp. 87-103 [doi: 10.1016/j.artmed.2009.07.001]).

The main result of the latter approach is generic query specification in domain-specific medical area. Queries are transformed to SQL, while at the same time admitting the fact that SQL does not provide a sufficiently efficient implementation in all cases.

There are also a lot of business intelligence tools which offer rich capabilities of analysing data in many dimensions as described in the paper of Schlegel, K. et al (Schlegel, K., Sallam, R.L., Yuen, D., Tapadinhas, J.: Magic Quadrant for Business Intelligence and Analytics Platforms, Gartner, February 2013).

International patent application publication No. PCT/US2012/031052 discloses a method of transacting medical information that includes receiving medical information from a medical sources, identifying, mapping, and consolidating the received medical information by a back-end medical processor, providing access to specific relevant data, based on a user's security privileges, within the identified, mapped, and consolidated medical information, based on user-specific functions or roles by a front-end medical processor, and generating user-customized processed medical information to a plurality of users, with at least a portion of the user-customized processed medical information being provided to each of the plurality of users based on its relevancy to each user's specific function or role and each user's associated security privileges.

United States of America patent publication No. US 8,417,727 discloses a method for storing data in a relational database system using a processor, wherein a collection of values is assigned to a structure dictionary, wherein each of the values represents the value of a row for an attribute and has a unique ordinal number within the collection, and wherein the structure dictionary contains structures defined based on at least one of interaction with a user of the system via an interface, automatic detection of structures occurring in data, and predetermined information about structures relevant to data content that is stored in the system.

Chinese patent application publication No. CN101986333 discloses an auxiliary decision supporting system of a hospital. The system comprises a production system layer, an interface layer, a data layer and an application layer. The system is characterized in that: the production system layer integrates data of each service system of the hospital, the data are integrated by the interface layer and organized to a data warehouse, the data required corresponding to each system of the application layer are extracted from the data warehouse through the data layer to form a data mart, and finally the data mart provides data support for the corresponding system so as to ensure development and operation of the corresponding system of the application layer.

United States of America patent publication No. US 8,898,798 discloses a system and method which enforces discipline in the clinical decision making process by ensuring proper and specific diagnoses and documentation thereof. This system supports the diagnosis, reports patient progress, enhances continuity of care through enhanced communication and notification throughout the treatment episode with all clinical and operational providers, automates core measure compliance documentation and ensure proper notification of any program specific risk management initiatives.

United States of America patent publication No. US 7,987,102 discloses a method and system that addresses the need for utilization analysis and performance evaluation of providers in a health care organization. The health care data comprising health plan information of consumers, providers, claims, and hospitals is collected, translated, and organized in a structured relational format and stored in standard tables. The organized health care data is analysed by calculating consumer statistics for a health plan using the health plan information of the consumers.

European patent application publication No. EP 2407092 discloses a system for capturing and maintaining a categorized flow of data in a clinical environment includes a distributed computer network, a plurality of data clusters dispersed over the network, a repository adapted to store data representative of the identity of patients, means for querying the repository to identify one of the data clusters associated with a particular patient and in response to such identification of the data cluster, allowing said particular patient or a user to query clinical data associated with the patient from said identified data cluster.

United States of America patent publication No. US 8,898,798 discloses a medical information navigation engine useful in association with at least one electronic health record system. The engine decouples identifying information from clinical data from electronic health records. The clinical data includes clinical narrative having discrete data and textual data. The identifying information is stored. Additionally, the identifying information is associated with a token in the clinical data. The clinical data may then be indexed. The discrete data and the textual data in the clinical data may then be mined. Mining includes extracting at least one relevant event from the discrete data and the textual data. Next, the clinical data and identifying information may be reintegrated using the token. The event associated with the mined discrete data and textual data may then be exported.

However, these tools do not concentrate on the *ad hoc* querying, but instead offer a plenty of features for data analysts to build custom, very complex reports and data visualizations. Thus, end-users still have only predefined capabilities and are not able to formulate *ad hoc* questions themselves.

Additionally, to the above mentioned problems, aim of the invention is to create a system and method for instantaneous *ad hoc* querying of hospital information. To consider a system and method to be instantaneous it should be able to respond to a query in less than one second having a database of millions to tens of millions of records.

<u>Summary of the Invention</u>

Aim of the invention is reached by developing a method and a system for instantaneous *ad hoc* querying of hospital information.

The present invention describes a method how to implement, in a hospital, a system allowing for instantaneous, also called easy and fast, *ad hoc* querying of hospital information. In this way, the following problem of administration and management in large hospitals is solved: to take their decisions, senior managers need obtaining quick answers to a large number and a great variety of successive *ad hoc* queries to the hospital information system. The desired response time - less than one second. The typical size of the database - millions to tens of millions of records. To save time, no programmers should be involved as mediators. It give possibility to correct quickly a mistaken query and quickly obtain an answer to it.

The present invention, first, proposes to apply a new method of database organization that is especially well suited for the specifics of hospital information - the so-called *granular databases.* This principle allows for fast processing of the queries, hospital managers are interested in. If necessary, a granular database can be distributed easily over multiple servers. Since real databases and data warehouses are not granular, to obtain a granular database, a specific *transformation method* should be applied. Secondly, the present invention proposes principles of ontology based, hospital management specific query languages, allowing for easy and adequate *ad hoc* query formulation by hospital managers, who are trained in working with *Excel*-type tables, but not in SQL programming. Programmers are no more needed as mediators. These principles of query language design ensure also easy distributing of query processing over multiple servers. And finally, the present invention proposes a new combination of methods allowing to implement efficiently the above-mentioned database organization and query processing (in a multi-server environment, if needed).

A system for *ad hoc* querying of hospital information comprises a database configured to store a data about patients and manipulations in the hospital, an input device, an output device and a processor. The database is further configured to store the data into granules, wherein each granule comprises a data related to a single patient. The database further comprising an in-memory database. The database can comprise a server or multiple servers in which granules are distributed. The input device is connected to the processor and configured to input a query in order to retrieve an answer. The output device is connected to the processor and configured to report to the user the answer. The processor is configured to receive the query from the input device and to retrieve the answer to the query processing granules stored in the database, wherein the answer is forwarded to the output device.

The system further comprises an interface configured to represent the query to the system entered by the user and display the answer received from the output device to the user. The system further comprises a query translation engine configured to translate a query language into sequences of applications of the API functions. The system further comprises a query processing engine configured to process the queries translated by the query translation engine in order to output a queried answer.

A method for execution on above described system for *ad hoc* querying of hospital information comprises the steps of:

- collecting and storing data from a database of a hospital information system as a set of text files in CSV format;
- re-designing the database schema of the hospital information system to obtain a granular ontology,
- storing information of the redesigned database as a text file in XML format;
- granulating data according to a granular ontology;
- loading granulated data into an in-memory database;
- generating for the granular ontology a library of API functions with a predetermined patterns;
- developing a query language according to the granular ontology and API functions;
- translating query language into sequences of applications of the API functions by means of query translation engine; and
- processing of queries translated by query translation engine by means of query processing engine in order to give a queried answer.

CSV is abbreviation of Comma-Separated Values file that stores tabular data such as numbers and text in plain text. Another term used in this application is API, which is abbreviation of Application Programming Interface. In computer programming, it is a set of routines, protocols, and tools for building software applications.

A method further includes the step of developing a query language according to the granular ontology and API functions includes a use of set expressions comprising

[SELECT] A X: F(X),

where A - class name, X is instance variable, and F(X) is Boolean expression for X.

A method further includes the step of developing a query language according to the granular ontology and API functions includes a use of expressions selected from the following groups of expressions:

- boolean expressions comprising
  {EXISTS, FORALL} set expression,
  {AND, OR, NOT} boolean expressions,
  {=, <, <= etc.} scalar expressions,
  attribute references;
- numeric expressions comprising
  COUNT set expression,
  {MIN, MAX, SUM, AVG} numeric expression, set expression,
  {+, -, *, /} numeric expressions,

numeric constants,
attribute references;

- string expressions comprising
SUBSTRING string expression, numeric expressions,
string constants,
attribute references;

- date/time expressions comprising
{date/time functions} date/time expressions,
date/time constants,
attribute references;

- table expressions comprising
SELECT A X: F(X),
BUILD TABLE expression1[X] (column_name1) expression2[X] (column_name2), SORT BY column_name,
SHOW FIRST n ROWS.

The present invention comprises a computer program product comprising software code for performing the operations of a system and a method when executed on a digital data processor.

In the present invention a new method of database organization is selected that is especially well suited for the specifics of hospital information - the so-called *granular databases.* This principle allows for fast processing of the queries, hospital managers are interested in. If necessary, a granular database can be distributed over multiple servers. Granularity provides a very efficient query implementation without involving SQL databases. In order to use the query language, data should be transformed to a granular database. Granular database contains data accordingly to a granular ontology. Thus data become granular and can be distributed across multiple databases in order to facilitate the execution of *ad hoc* queries. The query engine receives a query and retrieves the answer efficiently from the distributed granular databases. Real databases and data warehouses are not granular. To obtain a granular database, a specific transformation method is necessary.

The necessary transformation method is proposed in the paper of J. Barzdins et. al. (J. Barzdins, E. Rencis and A. Sostaks. Fast Ad Hoc Queries Based on Data Ontologies. Frontiers in Artificial Intelligence and Applications, Vol. 270, Databases and Information Systems VIII, IOS Press, pp. 43-56, 2014). Transformation is performed on the ontologies of databases. According to its ontology, the database contains instances of classes (objects) and instances of associations (links). Ontologies are represented usually as UML class diagrams, where classes are depicted as boxes, and associations - as arrows.

As a rule, associations are navigable in both directions. For example, if R is an association between classes A and B, then, as a rule, for any instance a1 of A, one can find quickly all instances of B which are connected to a1 via R. And conversely, for any instance b1 of B, one can find quickly all instances of A which are connected to b1 via R.

But sometimes, navigation via some association is supported only in one of the directions. Usually, such associations are implemented as simple foreign keys. For example, if R is an association between classes A and B, navigable only from A to B, and each instance of A is connected to a single instance of B, then, usually, an instance of A contain an identifying code of the corresponding instance of B as one of its properties (foreign key).

If, in an ontology, for some class C, all its associations are navigable only to C (and never - in the opposite direction), then such a class is called a classifier. Instances of C "classify" instances of the entities, connected to C. All the other classes in the ontology are called logical classes.

The transformation method is based on dividing of database classes into these two types: classifiers and logical classes. If some class C is selected to function as a classifier, then all associations to C are re-implemented in the partner classes as simple foreign keys, pointing to C, and the arrows of these associations are removed from the class diagram. Thus, the box representing a classifier, is detached from the rest of the class diagram. This selection process of classifiers is continued until the class diagram becomes a tree (or, equivalently, a star), consisting of logical classes only. The root of the tree (the centre of the star) is called the master class.

Every instance of any logical class is connected (via associations of the tree) to a single instance of the master class. The set of all instances connected to some instance of the master class, is called a granule. In hospital databases, usually, each granule will contain data related to a single patient.

At the implementation level, the application of the above-mentioned method means the following:

**Step 1.** Explore the database schema of the hospital information system.

**Step 2.** Extract the necessary part of the data (for example, by means of a sequence of SQL queries) and represent it as a set of easy readable files (for examples, as tables in CSV format).

**Step 3.** Re-design (as described above) the database schema to obtain a granular ontology, and store it in some readable text format.

**Step 4.** Implement a special data loading program allowing to load the data from the above-mentioned easy readable files into an in-memory database organized according to the ontology obtained in Step 3 (it could be implemented, for example, as a network of Java objects). If necessary, the granules of the in-memory database can be distributed over multiple servers.

The next component of the present invention are principles (a set of typical patterns) of ontology based query languages suited specifically for hospital managers. Query languages designed according to these principles allow for easy and adequate ad hoc query formulation by hospital managers, who are trained in working with Excel type tables but not in SQL programming. Programmers are no more needed as mediators.

In the result of extensive experiments with real hospital managers trained in working with Excel type tables but not in SQL programming, the following typical patterns of ad hoc queries (specific for hospital management) were selected, tested and recognized as sufficient:

set expressions
[SELECT] A X: F(X)
A - class name, X - instance variable, F(X) - Boolean expression for X
Selects all instances X of class A for which F(X) is true.

Since granular ontologies are tree-like, in queries, association names are obsolete and are omitted at all. In this way, preparing and understanding of queries becomes much easier.

If A X: F(X) is used as a sub-expression of some expression E, then one can use in attribute references:

a) attributes of the *current* instance X of class A;
b) attributes of the *current* instances of classes located in the expression E *above* the sub-expression;
c) attributes of those instances of other classes that are reachable in a unique way from the instances a), b) via associations of the ontology.

In this simple way, great flexibility and ease of preparing and understanding of queries is obtained.

Additionally, the following expressions are used: scalar expressions, Boolean expressions, numeric expressions, string expressions, date/time expressions.

Boolean expressions include the following:

{EXISTS, FORALL} set expression
{AND, OR, NOT} Boolean expression(s)
{=, <, <= etc.} scalar expressions
attribute references

Numeric expressions include the following:

COUNT set expression
{MIN, MAX, SUM, AVG} numeric expression, set expression
{+, -, *, /} numeric expressions
numeric constants
attribute references

String expressions include the following:

SUBSTRING string expression, numeric expression(s)
string constants
attribute references

Date/time expressions include the following:

{date/time functions} date/time expression(s)
date/time constants

attribute references

Table expressions include the following:

```
SELECT A X: F(X)
BUILD TABLE expression1[X] (column_name1) expression2[X] (column_name2) ...
SORT BY column_name
SHOW FIRST n ROWS
```

Rows of the table correspond to the selected instances of class A represented by the variable X.

The final component of the invention includes a method for efficient processing of the above types of queries over a (possibly distributed) granular database.

The efficiency of the query execution has been an important aspect of implementation of query languages, because of obvious reasons - the result of query have to be obtained in reasonable time. It is conventional that the linear time of execution of an algorithm regarding the size of data is acceptable. By applying the analysis methods represented in the paper of Janis Barzdins, et.al. (Juris Barzdins, Edgars Rencis, and Agris Sostaks. Graphical modelling and query language for hospitals. Health Information Science and Systems 2013, 1:14) it can be shown that, by applying a rather straightforward algorithm, the execution time of the above-mentioned types of queries over a distributed granular database can be made *linear* regarding the total number of instances in the database.

The main idea is to go through all the granules in the database and check all instances in the particular granule. If the condition evaluates to true, then the granule is added to the filtered dataset. Checking of a single granule does not require more time than some constant $C_{gran}$, thus the total time needed to evaluate a single condition on a single server granular database is $C_{gran}\ g$, where g is the number of granules in the database.

In the case of multiple servers, a good evaluation time of a single condition can be achieved by applying the following principles:

a) the necessary number of servers should be calculated in such a way that when processing a single condition, each server returns a table containing no more than a limited number of rows (for example, less than 1 megabyte);
b) classifier data are limited in size (tens of megabytes), so an entire copy of these data can be held on each server;
c) for merging of the responses, servers should be organized in a binary tree structure.

In this way, the evaluation time of a single condition can be reduced to

$$C_{gran}\frac{g}{s}+\left(C_{exch}+C_{merge}\right)\log s\,,$$

where:

s - the number of servers;
g - the total number of granules in the database;
$C_{gran}$ - the time necessary to check a single granule;
$C_{exch}$ - the time, necessary to exchange data between two servers;
$C_{merge}$ - the time, necessary to merge the data produced by two servers.

The principle (a) allows to keep the constants $C_{exch}$, $C_{merge}$ low. The principle (b) allows to retain the value of $C_{gran}$ calculated for the case of a single server. The principle (c) allows to use the multiplier log s instead of s.

**Step 5.** To speed up query processing, develop (or acquire) a universal program allowing to generate, for each granular ontology, automatically, a library of API functions. There are two kinds of functions: a) the global ones, generated for each ontology class C (these functions are scanning all instances of C); b) the local ones, generated for each pair C, D of ontology classes (these functions are scanning all instances of C reachable from a particular instance d of the class D). As parameters of functions lambda-expressions are used to define conditions for selection and values to be computed.

For example, the function sumC(valFn<C>, pred<C>) is scanning all instances of the class C, and is summing up values of valFn<C> of the instances satisfying the condition pred<C>. The function sumCD(d, valFn<C>, pred<C>) is scanning all instances of the class C, reachable from the instance d of the class D, and is summing up values of valFn<C> of the instance satisfying the condition pred<C>.

Apply the above-mentioned API library generation program to the ontology obtained in Step 3, and store the result.

The library of the API functions can have the following patterns:

| | |
|---|---|
| countC(pred<C>) | countCD(d, pred<C>) |
| sumC(valFn<C>, pred<C>) | sumCD(d, valFn<C>, pred<C>) |
| avgC(valFn<C>, pred<C>) | avgCD(d, valFn<C>, pred<C>) |
| maxC(valFn<C>, pred<C>) | maxCD(d, valFn<C>, pred<C>) |
| minC(valFn<C>, pred<C>) | minCD(d, valFn<C>, pred<C>) |
| mostC(valFn<C>, pred<C>) | mostCD(d, valFn<C>, pred<C>) |
| countDistinctC(valFn<C>, pred<C>) | countDistinctCD(d, valFn<C>, pred<C>) |
| findC(pred<C>) | findCD(d, pred<C>) |
| findOneC(pred<C>) | findOneCD(d, pred<C>) |
| anyC(pred<C>) | anyCD(d, pred<C>) |
| allC(pred<C>) | allCD(d, pred<C>). |

Above mentioned templates of the navigational API functions can be generated automatically for a granular ontology. There are two kinds of functions: a) the global ones, generated for each ontology class C (the functions are scanning all instances of C); b) the local ones, generated for each pair C, D of ontology classes (the functions are scanning all instances of C reachable from a particular instance d of the class D).

**Step 6.** For the granular ontology obtained in Step 3, develop a *query language* according to the above-stated principles. For ease of use, the idea of controlled natural languages (CNL) could be applied. For example, consider the following query: count *Patients* having *Episode* having *Operation* with *operationDuration*>60min

**Step 7.** Develop a *translation program* (compiler) allowing to convert queries (for example, CNL queries) of the Step 6 into sequences of applications of the API functions obtained in Step 5. For example, translate the above CNL query into the following one:

$$\_result = countPatient(pat\text{-}>anyEpisodePatient(pat, epi\text{-}>anyOperationEpisode$$

$$(epi, oper\text{-}>operationDuration>60)))$$

Here, *countPatient, anyEpisodePatient, anyOperationEpisode* are API functions, *operationDuration* is a derived attribute of *Operation,* and *pat, epi, oper* are temporary variable names denoting instances of the corresponding classes in the *lambda-expressions* generated. Nesting of lambda-expressions is allowed.

**Step 8.** Develop a query processing program for the queries generated by the program of Step 7 and the in-memory (possibly, multi-server) database obtained according to Step 4.

**Step 9.** Develop an application allowing the user to enter conveniently queries in the language of Step 6 and obtain the results of them. At start, the application should invoke the data loading program of Step 4 to build an in-memory granular database from the text files extracted from the hospital information system. Queries entered by the user are, first, translated by the program of Step 7, and after this, processed (possibly, on multiple servers) by the program of Step 8.

In the result of extensive experiments with real hospital data, it has been established, that the an optimized implementation of the proposed solution works acceptably on 64 bit, 4 gigabyte RAM Windows desktop or laptop computers with databases containing data up to 60000 patients. This corresponds to the yearly number of patients treated in a medium size hospital in, for example, Latvia (500 beds in the stationary block plus the block of ambulatory services).

For larger hospitals the necessary performance can be achieved by distributing the granules over multiple servers and by applying the above-mentioned principles (a, b, c). Queries corresponding to the above-mentioned patterns allow for easy distributing of their processing over multiple servers.

Brief description of the drawings

The following disclosure will be better understood by a person skilled in the art when read in conjunction with the figures, which show example embodiments. The figures are for the purpose of illustrating example system and method, but it is understood that the various inventions, described herein, are not limited to the arrangements and instrumentality shown in the figures.

Fig. 1 shows an overall architecture of system;
Fig. 2 shows an ontology of a simplified hospital information database before transforming it to a granular database;
Fig. 3 shows an ontology of a simplified hospital information database after transforming it to a granular database;
Fig. 4 shows the ontology of real hospital information database after transformation to a granular database; An example of a graphical representation of such an ontology is shown in Fig. 4, it corresponds to a database of a real medium size hospital in Latvia.

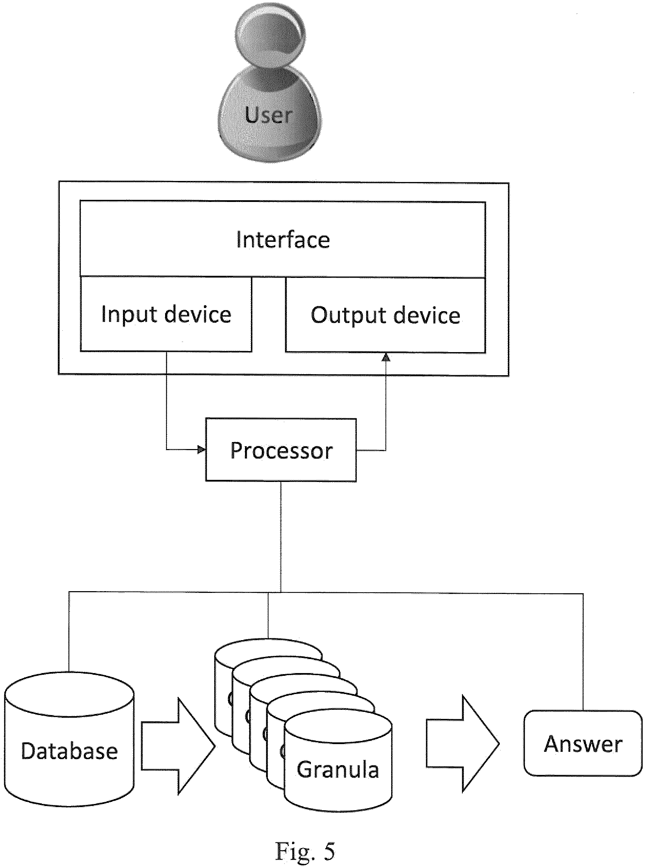

Fig. 5 is a block diagram that illustrates a system ad hoc querying of hospital information.

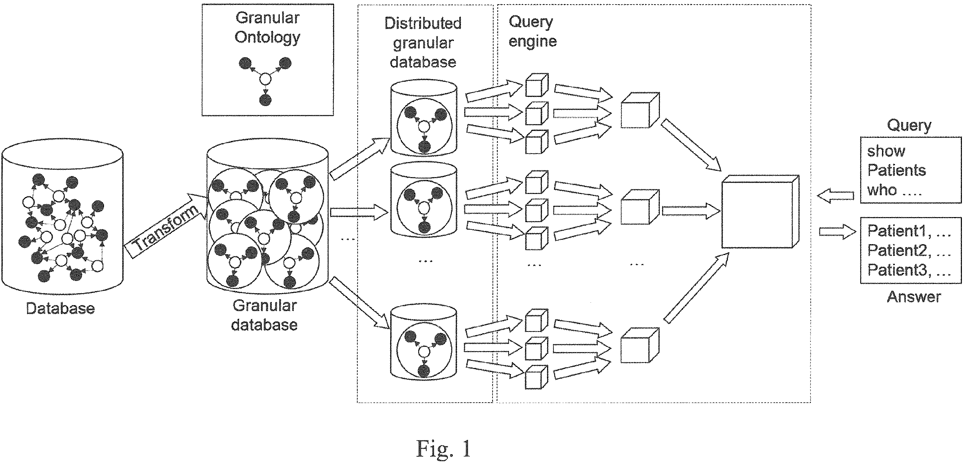

Fig. 1 shows the overall architecture of a fast *ad hoc* querying system. In order to use the query language, data should be transformed to a granular database. Granular database contains data accordingly to a granular ontology. Thus data become granular and can be distributed across multiple databases in order to facilitate the execution of *ad hoc* queries. The query engine receives a query and retrieves the answer efficiently from the distributed granular databases.

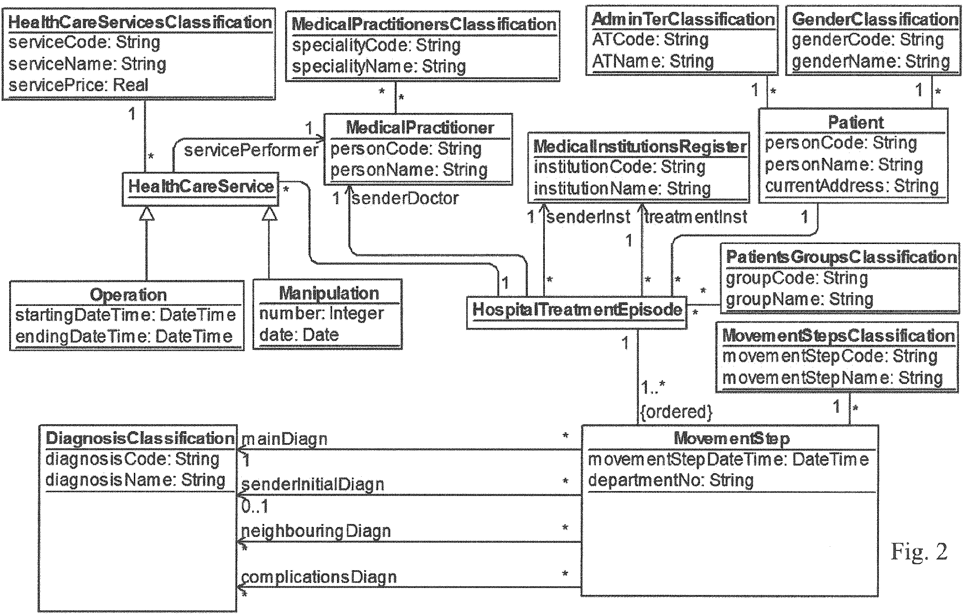

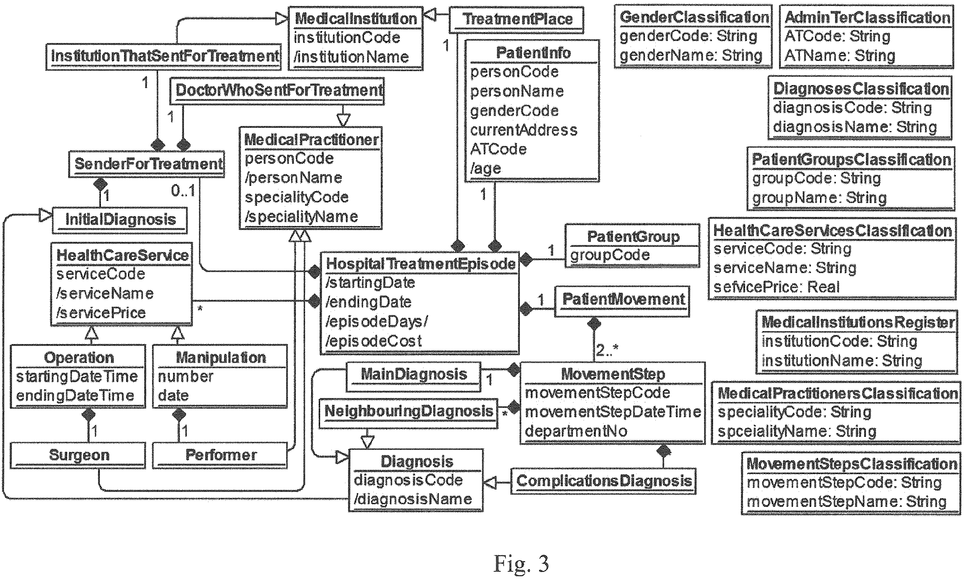

Fig. 2 shows an ontology of a simplified hospital database before transforming it to a granular database. The boxes depicted in yellow represent the candidate-entities for redefining as classifiers. Fig. 3 shows an ontology of a simplified hospital database after transforming to a granular database. The boxes depicted in yellow in the bottom part of the drawing represent the entities redefined as classifiers.

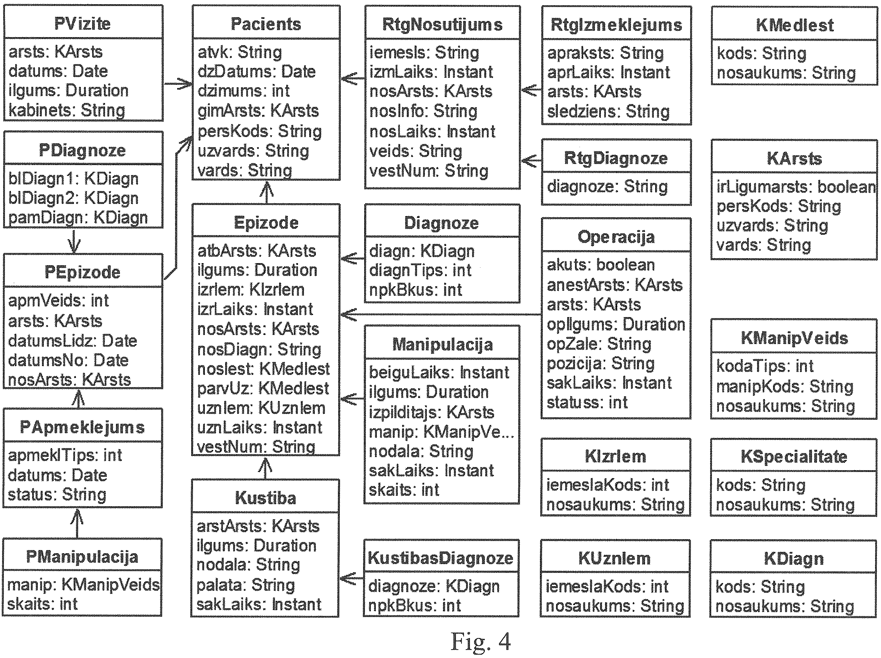

Fig. 4 shows the ontology of a real hospital database after transformation to a granular database. This structure was used in the first embodiment of a fast *ad hoc* querying system designed according to the principles of the present invention. According to the real situation, classes and attributes are named in Latvian. Compare with the simplified, but similar Fig. 3 where names are given in English.

The master class of the ontology is *Pacients* (personal data of a patient). The main classes of the next level are: *Epizode* (hospital treatment episode - in the stationary block) and *PEpizode* (hospital treatment episode - in the block of ambulatory services). The main classes of the third level: *Kustiba* (patient movement - in the stationary block), *PApmeklejums* (patient's visit of the ambulatory block).

The description of the preferred embodiment of the present invention is not intended to be exhaustive or limit the invention in the form disclosed. Many variations will be apparent to those of ordinary skill.

The first really usable embodiment of the proposed method and system was implemented for a medium size hospital in Latvia. It was implemented as a desktop Java application running on 64 bit Windows desktops or laptops with at least 4 gigabyte RAM. Hardware of this size will be sufficient for processing of up to 60 000 patients 1 year hospital data. Each hospital manager interested in a fast *ad hoc* querying of hospital data should install the application on his/her own workstation. For larger amounts of data a client-server version should be implemented, with multiple client workstations connected to the internet, and if necessary, with multiple servers.

The implemented application is working with a read-only in-memory database organized according to the ontology shown in Fig.4 and implemented as a network of Java objects. The in-memory database is generated from a specially prepared set of text files in CSV format (see Step 2 below) each time when the application is starting.

During the implementation, the following steps were performed according to the proposal above:

**Step 1.** The system was implemented for a medium size hospital in Latvia (treating about 60 000 patients yearly, 500 beds in the stationary block plus the block of ambulatory services). The POSTGRES database schema of the hospital information system was analysed.

**Step 2.** The part of the data necessary for the annual reporting was extracted by means of a sequence of SQL queries and stored as a set of text files (tables in CSV format).

**Step 3.** The database schema was re-designed to obtain a granular ontology. The resulting ontology was stored as a text file in XML format. The corresponding graphical representation of the ontology is shown in Fig. 4.

**Step 4. A** special data loading program was implemented in Java, allowing to load the data from the text files in CSV format (Step 2) into an in-memory database organized as a network of Java objects according to the ontology shown in Fig. 4 (Step 3).

**Step 5.** A universal program was developed in Java, allowing to generate for each granular ontology, automatically, a *library of API functions.* The program was applied to the ontology shown in Fig. 4.

**Step 6.** For the granular ontology shown in Fig. 4, a *query language* was developed according to the above-stated principles. For ease of use, the idea of controlled natural languages (CNL) was applied. Some examples of queries in this language are as follows:

"COUNT kustibam, WHERE nodala=12-69
COUNT kustibam, WHERE nodala=12-69 AND npk=* AND epizode.izrIem.iemeslaKods=3

COUNT epizodem, WHERE (kustiba, WHERE npk=*).nodala=12-69 AND izrIem.iemeslaKods=3
COUNT epizodem, WHERE (kustiba, WHERE npk=* AND arstArsts.vards<>Arta).nodala=12-69 AND izrIem.iemeslaKods=3
COUNT kustibam k, WHERE k.nodala=12-69 AND next(k).nodala=12-60
COUNT kustibam, WHERE nodala=12-69 AND npk>1
COUNT epizodes WHERE izriem.iemeslakods=3 AND (uznemsanasdiagnozes, WHERE diagntips=2).skaits>1 AND EXISTS kustiba WHERE nodala=12-69 AND npk=*
COUNT kustibam, WHERE nodala=12-69 AND npk=* AND epizode.parvUz.kods<>null
COUNT epizodem, WHERE izriem.iemeslakods=2 AND EXISTS kustiba, WHERE nodala=12-69 AND npk=*
SHOW ALL epizodi, WHERE EXISTS kustiba, WHERE nodala=12-69, SHOW vesturesNum, nosDiagn
SHOW ALL uznemsanasDiagnozes, WHERE diagntips=2 AND EXISTS kustiba, WHERE nodala=12-69, SHOW epizode.vesturesNum, npkBkus, diagnoze.kods, diagnoze.nosaukums
COUNT kustibas, WHERE nodala=12-69 AND EXISTS KustibasDiagnoze, WHERE diagnoze.kods.substring(1,1)=A OR diagnoze.kods.substring(1,1)=B
COUNT kustibas, WHERE nodala=12-69 AND EXISTS uznemsanasdiagnoze, WHERE diagntips=2 AND (diagnoze.kods.substring(1,3)>=A00 AND diagnoze.kods.substring(1,3)<=A09)
COUNT kustibas, WHERE nodala=12-69 AND npk=* AND epizode.izrIem.iemeslaKods=3 AND EXISTS UznemsanasDiagnoze, WHERE diagnTips=2 AND (diagnoze.kods.substring(1,1)=A OR diagnoze.kods.substring(1,1)=B)
COUNT kustibas, WHERE nodala=12-69 AND EXISTS KustibasDiagnoze, WHERE diagnoze.kods.substring(1,1)=C AND NOT EXISTS UznemsanasDiagnoze, WHERE diagnTips=2 AND diagnoze.kods.substring(1,1)=C
COUNT kustibas, WHERE nodala=12-69 AND EXISTS UznemsanasDiagnoze, WHERE diagnTips=2 AND diagnoze.kods.substring(1,3)>=D50 AND diagnoze.kods.substring(1,3)<=D89
COUNT kustibas, WHERE nodala=12-69 AND npk=* AND epizode.izriem.iemeslakods=3 AND EXISTS uznemsanasdiagnoze, WHERE diagntips=2 AND (diagnoze.kods.substring(1,1)=A OR diagnoze.kods.substring(1,1)=B) COUNT manipulacijam, WHERE manipVeids.kodaTips=2 AND (manipVeids.manipKods=04142 OR manipVeids.manipKods=04144)
FIND IN INTERVAL (1-12) ALL VALUES x, radit x, (COUNT manipulacijam, WHERE sakumaLaiks.month=x AND manipVeids.kodaTips=2 AND (manipVeids.manipKods=04142 OR manipVeids.manipKods=04144))
COUNT manipulacijam, WHERE sakumaLaiks.month=2 AND manipVeids.kodaTips=2 AND (manipVeids.manipKods=04142 OR manipVeids.manipKods=04144)
FIND IN INTERVAL (1-12) ALL VALUES x, radit x, (COUNT manipulacijam, WHERE sakumaLaiks.month=x AND manipVeids.kodaTips=2 AND manipVeids.manipKods=04142) (tikai vienai manipulacijai)
FIND IN INTERVAL (1-12) ALL VALUES x, SHOW x (COLUMN Menesis), (COUNT manipulacijas WHERE manipveids.kodatips=2 AND manipveids.manipkods=18073 AND sakumalaiks.month=x) (COLUMN Tonsil), (COUNT manipulacijas WHERE manipveids.kodatips=2 AND manipveids.manipkods=18079 AND sakumalaiks.month=x) (COLUMN Adenot)
COUNT kustibas, WHERE nodala=12-69 AND EXISTS uznemsanasdiagnoze, WHERE diagntips=2 AND (diagnoze.kods.substring(1,1)=A OR diagnoze.kods.substring(1,1)=B) SHOW ALL kustibas WHERE epizode.izriem.iemeslakods=3 AND nodala=12-69 AND npk=*
SHOW ALL epizodes WHERE izriem.iemeslakods=3 AND EXISTS kustiba WHERE nodala=12-69 AND npk=*
FIND ALL epizodes x, WHERE izriem.iemeslakods=3, MAKE TABLE x.vesturesnum (COLUMN A), (kustiba, WHERE npk=*).nodala (COLUMN B), (uznemsanasdiagnoze, WHERE diagntips=3).diagnoze.kods (COLUMN c), (uznemsanasdiagnoze, WHERE diagntips=3).diagnoze.nosaukums (COLUMN d)
FIND ALL epizodes x, MAKE TABLE x.vesturesnum (COLUMN A), (uznemsanasdiagnoze, WHERE diagntips=1 AND npkbkus=1).dianoze.nosaukums (COLUMN B)

FIND Kustibam ATTRIBUTE nodala ALL atsķirīgas VALUES x, SHOW x (COLUMN Nodala), (COUNT Kustibas WHERE nodala =x)(COLUMN Skaits)

FIND Kustībām ATTRIBUTE nodala ALL atšķirīgas VALUES x, SHOW x (COLUMN Nodala), (COUNT Kustības WHERE nodala =x)(COLUMN Skaits), SORT DESCENDING pec COLUMN Skaits

FIND ALL operācijas WHERE beiguLaiks-sakumaLaiks > 2st30min, SHOW sakumalaiks, beigulaiks, opZale, anesteziologs.uzvards

FIND IN INTERVAL (1-12) ALL VALUES x, SHOW x (COLUMN Menesis), (COUNT manipulacijas WHERE sakumalaiks.month=x) (COLUMN Skaits)

FIND IN INTERVAL (1-12) ALL VALUES x, MAKE TABLE x (COLUMN Menesis), (COUNT manipulacijas, WHERE manipveids.kodatips=3 AND (manipveids.manipkods.substring(1,1)=P OR manipveids.manipkods.substring(1,1)=F) AND sakumalaiks.month=x) (COLUMN Skaits)

FIND ALL operacija, SHOW epizode.vesturesNum (COLUMN F), (sakumaLaiks - epizode.uznemsanaslaiks) (COLUMN C), (epizode.izrakstisanasLaiks - beiguLaiks) (COLUMN Z), (uznemsanasdiagnoze, WHERE diagnTips=3).diagnoze.nosaukums (COLUMN a), arsts.uzvards ".

**Step 7.** A *translation program* was developed in Java, allowing to convert CNL queries in the language developed in the Step 6 into applications of the library API functions obtained in Step 5.

**Step 8.** A query processing program was developed in Java for processing of queries generated by the program of Step 7 and the in-memory database obtained according to Step 4.

**Step 9.** An application was developed in Java, allowing the user to enter conveniently CNL queries in the language of Step 6 and obtain the results of them. At start, the application invokes the data loading program of Step 4 to build an in-memory granular database from the text files extracted in Step 2 from the hospital information system. Queries entered by the user are, first, translated by the program of Step 7, and after this, processed by the program of Step 8.

While the inventions have been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from the spirit or scope of the present inventions. Therefore, it is intended that the inventions not be limited to the particular embodiments disclosed herein.

**Claims**

1. A system for instantaneous *ad hoc* querying of hospital information, wherein the system comprises:

   a database configured to store a data about patients and manipulations in the hospital;
   a database is further configured to store the data into granules, wherein each granule comprises a data related to a single patient;
   an input device connected to a processor and configured to input a query in order
   to retrieve an answer;
   an output device connected to a processor and configured to report to the user the answer; and
   a processor configured to receive the query from the input device and to retrieve the answer to the query processing granules stored in the database, wherein the answer is forwarded to the output device.

2. A system according to Claim 1, wherein it further comprises an interface configured to represent the query to the system entered by the user and display the answer received from the output device to the user.

3. A system according to Claim 1 or 2, wherein database further comprising an in-memory database.

4. A system according to any Claim 1 to 3, wherein the system further comprises a query translation engine configured to translate a query language into sequences of applications of the API functions.

5. A system according to any Claim 1 to 4, wherein the system further comprises a query processing engine configured to process the queries translated by the query translation engine in order to output a queried answer.

6. A system according to any Claim 1 to 5, wherein the database comprises a server or multiple servers in which granules are distributed.

7. A method, for execution on a system according to any preceding Claim, for *ad hoc* querying of hospital information, the method comprising the steps of:

- collecting and storing data from a database of a hospital information system as a set of text files in CSV format;
- re-designing the database schema of the hospital information system to obtain a granular ontology,
- storing information of the redesigned database as a text file in XML format;
- granulating data according to a granular ontology;
- loading granulated data into an in-memory database;
- generating for the granular ontology a library of API functions with a predetermined patterns;
- developing a query language according to the granular ontology and API functions;
- translating query language into sequences of applications of the API functions by means of query translation engine;
- processing of queries translated by query translation engine by means of query processing engine in order to give a queried answer.

8. A method for *ad hoc* querying of hospital information according to the Claim 7, wherein the step of developing a query language according to the granular ontology and API functions includes a use of set expressions comprising [SELECT] A X: F(X),
where A - class name, X is instance variable, and F(X) is Boolean expression for X.

9. A method for *ad hoc* querying of hospital information according to the Claim 7 or 8, wherein the step of developing a query language according to the granular ontology and API functions includes a use of expressions selected from the following groups of expressions:

- boolean expressions comprising
{EXISTS, FORALL} set expression,
{AND, OR, NOT} boolean expressions,
{=, <, <= etc.} scalar expressions,
attribute references;
- numeric expressions comprising
COUNT set expression,
{MIN, MAX, SUM, AVG} numeric expression, set expression,
{+, -, *, /} numeric expressions,
numeric constants,
attribute references;
- string expressions comprising
SUBSTRING string expression, numeric expressions,
string constants,
attribute references;
- date/time expressions comprising
{date/time functions} date/time expressions,
date/time constants,
attribute references;
- table expressions comprising
SELECT A X: F(X),
BUILD TABLE expression1[X] (column_name1) expression2[X] (column_name2),
SORT BY column_name,
SHOW FIRST n ROWS.

10. A computer program product comprising software code for performing the operations of a system according to Claims 1 to 6 when executed on a digital data processor.

11. A computer program product comprising software code for performing the operations of a method according to Claims 7 to 9 when executed on a digital data processor.

EP 3 131 026 A1

Fig. 1

**HealthCareServicesClassification**
serviceCode: String
serviceName: String
servicePrice: Real

**MedicalPractitionersClassification**
specialityCode: String
specialityName: String

**AdminTerClassification**
ATCode: String
ATName: String

**GenderClassification**
genderCode: String
genderName: String

1

servicePerformer

**MedicalPractitioner**
personCode: String
personName: String

1

**MedicalInstitutionsRegister**
institutionCode: String
institutionName: String

**Patient**
personCode: String
personName: String
currentAddress: String

**HealthCareService**

1 senderDoctor

1 senderInst    treatmentInst

1

**PatientsGroupsClassification**
groupCode: String
groupName: String

**Operation**
startingDateTime: DateTime
endingDateTime: DateTime

**Manipulation**
number: Integer
date: Date

**HospitalTreatmentEpisode**

**MovementStepsClassification**
movementStepCode: String
movementStepName: String

1

1..*

{ordered}

**DiagnosisClassification**
diagnosisCode: String
diagnosisName: String

mainDiagn

1

senderInitialDiagn

0..1

neighbouringDiagn

complicationsDiagn

**MovementStep**
movementStepDateTime: DateTime
departmentNo: String

Fig. 2

Fig. 3

**PVizite**

arsts: KArsts
datums: Date
ilgums: Duration
kabinets: String

**PDiagnoze**

blDiagn1: KDiagn
blDiagn2: KDiagn
pamDiagn: KDiagn

**PEpizode**

apmVeids: int
arsts: KArsts
datumsLidz: Date
datumsNo: Date
nosArsts: KArsts

**PApmeklejums**

apmeklTips: int
datums: Date
status: String

**PManipulacija**

manip: KManipVeids
skaits: int

**Pacients**

atvk: String
dzDatums: Date
dzimums: int
gimArsts: KArsts
persKods: String
uzvards: String
vards: String

**Epizode**

atbArsts: KArsts
ilgums: Duration
izrIem: KIzrIem
izrLaiks: Instant
nosArsts: KArsts
nosDiagn: String
nosIest: KMedIest
parvUz: KMedIest
uznIem: KUznIem
uznLaiks: Instant
vestNum: String

**Kustiba**

arstArsts: KArsts
ilgums: Duration
nodala: String
palata: String
sakLaiks: Instant

**RtgNosutijums**

iemesls: String
izmLaiks: Instant
nosArsts: KArsts
nosInfo: String
nosLaiks: Instant
veids: String
vestNum: String

**Diagnoze**

diagn: KDiagn
diagnTips: int
npkBkus: int

**Manipulacija**

beiguLaiks: Instant
ilgums: Duration
izpilditajs: KArsts
manip: KManipVe...
nodala: String
sakLaiks: Instant
skaits: int

**KustibasDiagnoze**

diagnoze: KDiagn
npkBkus: int

**RtgIzmeklejums**

apraksts: String
aprLaiks: Instant
arsts: KArsts
sledziens: String

**RtgDiagnoze**

diagnoze: String

**Operacija**

akuts: boolean
anestArsts: KArsts
arsts: KArsts
opIlgums: Duration
opZale: String
pozicija: String
sakLaiks: Instant
statuss: int

**KIzrIem**

iemeslaKods: int
nosaukums: String

**KUznIem**

iemeslaKods: int
nosaukums: String

**KMedIest**

kods: String
nosaukums: String

**KArsts**

irLigumarsts: boolean
persKods: String
uzvards: String
vards: String

**KManipVeids**

kodaTips: int
manipKods: String
nosaukums: String

**KSpecialitate**

kods: String
nosaukums: String

**KDiagn**

kods: String
nosaukums: String

Fig. 4

EP 3 131 026 A1

Fig. 5

## EUROPEAN SEARCH REPORT

Application Number

EP 15 18 0838

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Janis Barzdins ET AL: "Fast ad hoc queries based on data ontologies" In: "DATABASES AND INFORMATION SYSTEMS VIII", 31 December 2014 (2014-12-31), IOS Press, XP055242717, vol. 270, pages 43-56, DOI: 10.3233/978-1-61499-458-9-43, * page 43 - page 55 * | 1-11 | INV. G06F19/00 |
| A | Anonymous: "In-memory database - Wikipedia, the free encyclopedia", , 20 July 2015 (2015-07-20), XP055242746, Retrieved from the Internet: URL:https://en.wikipedia.org/w/index.php?title=In-memory_database&oldid=672199746 [retrieved on 2016-01-19] * page 1 * | 1-11 | |

TECHNICAL FIELDS
SEARCHED      (IPC)

G06F

The present search report has been drawn up for all claims

| Place of search | Date of completion of the search | Examiner |
|---|---|---|
| Munich | 20 January 2016 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012031052 W
- US 8417727 B
- CN 101986333
- US 8898798 B
- US 7987102 B
- EP 2407092 A

**Non-patent literature cited in the description**

- **PING, X.O. ; CHUNG, Y. et al.** A Web-Based Data-Querying Tool Based on Ontology-Driven Methodology and Flowchart-Based Model. *JMIR Medical Informatics,* 2013, vol. 1 (1
- **HUSER, V. ; NARUS, S.P. ; ROCHA, R.A.** Evaluation of a flowchart-based EHR query system: a case study of RetroGuide. *J Biomed Inform,* February 2010, vol. 43 (1), 41-50
- **AUSTIN, T. ; KALRA, D. ; TAPURIA, A. ; LEA, N. ; INGRAM, D.** Implementation of a query interface for a generic record server. *Int J Med Inform,* November 2008, vol. 77 (11), 754-764
- **MABOTUWANA, T. ; WARREN, J.** An ontology-based approach to enhance querying capabilities of general practice medicine for better management of hypertension. *Artif Intell Med,* October 2009, vol. 47 (2), 87-103
- **SCHLEGEL, K. ; SALLAM, R.L. ; YUEN, D. ; TAPADINHAS, J.** Magic Quadrant for Business Intelligence and Analytics Platforms. Gartner, February 2013
- Fast Ad Hoc Queries Based on Data Ontologies. **J. BARZDINS ; E. RENCIS ; A. SOSTAKS.** Frontiers in Artificial Intelligence and Applications, Vol. 270, Databases and Information Systems VIII. IOS Press, 2014, vol. 270, 43-56
- **JURIS BARZDINS ; EDGARS RENCIS ; AGRIS SOSTAKS.** Graphical modelling and query language for hospitals. *Health Information Science and Systems,* 2013, vol. 1, 14